(19) Europäisches Patentamt
European Patent Office
Office européen des brevets

(11) Veröffentlichungsnummer: **0 193 064**
**B1**

(12) # EUROPÄISCHE PATENTSCHRIFT

(45) Veröffentlichungstag der Patentschrift: **08.11.89**

(51) Int. Cl.⁴: **C 07 C 43/295**, C 07 C 41/26

(21) Anmeldenummer: **86101977.6**

(22) Anmeldetag: **17.02.86**

(54) **Verfahren zur Herstellung von 4,4'-Dihydroxidiphenylether.**

(30) Priorität: **27.02.85 DE 3506845**

(43) Veröffentlichungstag der Anmeldung: **03.09.86 Patentblatt 86/36**

(45) Bekanntmachung des Hinweises auf die Patenterteilung: **08.11.89 Patentblatt 89/45**

(84) Benannte Vertragsstaaten: **DE**

(56) Entgegenhaltungen:
**EP-A- 0 100 451**
**US-A- 3 290 386**
**US-A- 4 326 088**

**CHEMICAL ABSTRACTS, Band 75, Nr. 11, 13. September 1971, Seite 392, Spalte 1, Zusammenfassungsnr. 75782p, Columbus, Ohio, US; C.G. REID "Aromatic oxygenation with the peroxide-catalyst system. Peroxides-cupric chloride. Benzoyl peroxide-iodine", & DISS. ABSTR. INT. B 1970, 31(6), 3220**
**Houden-Weyl, Methoden der organischen Chemie, V/4, 1960, S. 519, 564, 566, 577**

(73) Patentinhaber: **Röhm GmbH, Kirschenallee Postfach 4242, D-6100 Darmstadt 1 (DE)**

(72) Erfinder: **Riemann, Achim, Dr., Hausweg 25, D-6103 Griesheim (DE)**
Erfinder: **Ude, Werner, Dr., Birngartenweg 115, D-6100 Darmstadt-Arheilgen (DE)**

**EP 0 193 064 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Gebiet der Erfindung

Die Erfindung betrifft ein Verfahren zur Herstellung von 4,4'-Dihydroxidiphenylether aus Diphenylether.

Stand der Technik

4,4'-Dihydroxidiphenylether mit der chemischen Formel

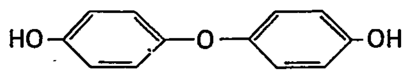

ist als Zwischenprodukt, insbesondere als bifunktionelle Verbindung für die Herstellung von Kunststoffen interessant. Einer technischen Verwendung dieser Substanz für die Herstellung von Polykondensationsharzen mit vorteilhaften Eigenschaften, z.B. von entsprechenden Polycarbonaten, stehen unbefriedigende Herstellungsverfahren entgegen.

Nach Kaloshin und Khvostov. Metody Poluch. Khim. Reaktiv. Prep. 1971. Nr. 23, 54–55 (Ref.: C.A. 78, 29358u) wird 4,4'-Dihydroxidiphenylether in einer Ausbeute von 51,5% durch Diazotierung von 4,4'-Diaminodiphenylether und anschließender Zersetzung des Diazoniumsalzes mit Schwefelsäure erhalten.

In der US-PS 3290386 wird ein Verfahren zur Herstellung von 4,4'-Dihydroxidiphenylether ausgehend von 4,4'-Dibromdiphenylether beschrieben, wobei dieser durch Natronlauge in Gegenwart von Kupfer(I)-ionen und Natriumperoxid bei 185 bis 190 °C hydrolysiert wird. Nach der Neutralisation mit Salzsäure wird das Verfahrenprodukt in nahezu quantitativer Ausbeute erhalten.

Bei dem Verfahren von Kaloshin und Khvostov muß der als Ausgangspunkt dienende Diaminosiphenylether über mehrere aufwendige Reaktionsstufen, zuletzt die Reduktion der entsprechenden Dinitroverbindung, hergestellt werden. Die Hydrolyse nach der US-PS liefert neben dem gewünschten Produkt als Beiprodukt Natriumbromid in wäßriger Lösung. Dieses und die bei der Herstellung der Ausgangsverbindung durch Bromierung von Diphenylether entstehende Bromwasserstoffsäure müssen entsorgt werden, was bei der hohen Korrosivsität und schwierigen Handhabung von Bromwasserstoff und gegebenenfalls dem daraus zurückgewonnenen Brom erhebliche Probleme verursacht.

Aufgabe und Lösung

Es bestand daher die Aufgabe, ein Verfahren zur Herstellung von 4,4'-Dihydroxidiphenylether zu finden, das von dem preisgünstigen Ausgangsprodukt Diphenylether ausgeht und Hilfsstoffe verwendet, die sich problemloser aufarbeiten lassen und wodurch das Verfahren insgesamt wirtschaftlicher und das Produkt erst technisch herstellbar ist.

Die Lösung dieser Aufgabe besteht darin

1. Diphenylether in den Para-Stellungen oxidativ, d. h. ohne die Bildung äquivalenter Mengen Halogenwasserstoff zu jodieren,
2. die Jodierungsprodukte mit Alkalihydroxid zu hydrolysieren,
3. durch Säurezugabe 4,4'-Dihydroxidiphenylether freizusetzen,
4. in dem sauren Filtrat das Jodid zu Jod zu oxidieren und
5. das dabei in fester Form anfallende und leicht abtrennbare Jod wieder in die Jodierung nach 1. zurückzuführen.

Aromatische Chlor- und Bromverbindungen lassen sich im allgemeinen leicht durch direkte Einwirkung der Halogene auf entsprechende Aromaten herstellen, wobei noch die äquivalente Menge Halogenwasserstoff entsteht. Die analoge Synthese aromatischer Jodverbindungen ist nicht möglich. Durch Verwendung von Natriumtrijodid gelingt nach EP-A 100 451 die Jodierung aromatischer Verbindungen, wobei theoretisch zwei Drittel des eingebrachten Jods für die Substitutionsreaktion ungenutzt bleiben. Diese Patentschrift beschreibt weiter noch die Hydrolyse der Jodverbindung durch deren Erhitzen mit wäßrigem Alkali in Gegenwart von metallischem Kupfer oder Kupfer-I-Salz als Katalysatoren.

Erst in Gegenwart von Oxidationsmitteln, wie z. B. Salpetersäure, Persulfaten, Jodsäure und auch Wasserstoffperoxid ist eine Jodierung aromatischer Verbindungen unter Ausnutzung des insgesamt eingebrachten Jods möglich (s. dazu Houben-Weyl, Methoden der organischen Chemie, Band V/4, 1960, Seiten 519, 564, 566 und 577). So beschreiben Wirth, Königstein und Kern, Ann. 634 (1960), 84, die Herstellung von 4,4'-Dijoddiphenylether in einer Reinausbeute von 71% d. Th. durch Einwirkung von Jod und Jodsäure auf Diphenylether. Eine Jodierungsvariante mit Bis-(trifluoracetoxi)jodbenzol und Jod als Jodierungssystem geben Merkushev, Simakhina und Koveshnikova. Synthesis 1980, 486, an, wobei 4,4'-Dijoddiphenylether in einer Ausbeute von 79% erhalten wird. Sowohl diese trivalente organische Jodverbindung als auch Jodsäure sind als Oxidationsmittel für einen Technischen Einsatz zu teuer.

Es wurde gefunden, dass man als Oxidationsmittel für die Herstellung von p-jodierten Diphenylethern durch Jodierung von Diphenylether vorteilhaft preiswerte anorganische Peroxoverbindungen, wie z. B. Ammoniumpersulfat bzw. Natriumpersulfat einsetzt, wobei 4,4'-Dijoddiphenylether in praktisch quantitativer Ausbeute gebildet wird oder die Jodierung in Gegenwart von Wasserstoffperoxid durchführt, wobei Gemische von 4,4'-Dijoddiphenylether und 4-Hydroxi-4'-Jod-diphenylether erhalten werden. Die direkte Jodierung von aromatischen Verbindungen, hier insbesondere von Diphenylether, mit Jod und Oxidationsmitteln, wobei die zu verwendenden Oxidationsmittel ein für die Oxidation von Jodwasserstoff zu Jod ausreichendes Oxidationspotential besitzen, soll hier als oxidative Jodierung bezeichnet werden.

Es wurde weiter gefunden, dass die Jodierung mit $H_2O_2$ als Oxidationsmittel neben der bekannten Säurekatalyse noch der Schwermetallkatalyse bedarf. Als Schwermetallkatalysatoren kommen hier solche in Betracht, die wie z. B. Kupfer, Eisen, Kobalt, in verschiedenwertigen Oxidationsstufen vorkommen und leicht in diese überführbar sind. In Abwesenheit von z. B. Kupfer wird unter sonst vergleichbaren Reaktionsbedingungen ein geringer Umsatz erzielt.

Weiter wurde gefunden, daß die Jodierungsprodukte 4,4'-Dijoddiphenylether und 4-Hydroxi-4'-Jod-diphenylether durch alkalische Hydrolyse quantitativ zu dem entsprechenden Diphenolat und Jodid umgesetzt werden, aus denen dann in bekannter Weise durch Säurezugabe 4,4'-Dihydroxidiphenylether freigesetzt und abfiltriert wird und im Filtrat nach technisch bekannten Verfahren, z. B. mit $C1_2$ oder $H_2O_2$, Jodid quantitativ zu Jod oxidiert wird, das in die Jodierungsstufe zurückgeführt wird.

Die Erfindung betrifft daher ein Verfahren zur Herstellung von 4,4'-Dihydroxidiphenylether aus Diphenylether durch lodierung des Diphenylethers und hydrolytischer Abspaltung des lods, dadurch gekennzeichnet, daß die lodierung des Diphenylethers in Gegenwart von Lösungsmitteln mit elementarem Jod und anorganischen Peroxoverbindungen als Oxidationsmittel in Gegenwart starker Säuren als Katalysatoren, wobei im Falle von $H_2O_2$ als Peroxoverbindung noch Katalysatoren von Schwermetallen, die in verschiedenen Oxidationsstufen vorkommen und leicht in diese überführbar sind, mitverwendet werden, bei Temperaturen von etwa 20 bis 100 Grad C durchgeführt wird, anschliessend der jodierte Diphenylether in wäßrig-alkalischem Medium in Gegenwart katalytischer Mengen von Kupfer bzw. Kupferionen und Alkali- bzw. Erdalkaliperoxid bei 100 bis 200 Grad C hydrolysiert wird und dann aus der filtrierten Lösung durch Säurezugabe unter Einstellung eines ph-Wertes $\leq 7$ der 4,4'-Dihydroxidiphenylether freigesetzt wird.

Vorteile der Erfindung

Die erfindungsgemäße Herstellung von 4,4'-Dijod- bzw. Hydroxi-Jod-diphenylethern durch oxidative Jodierung von Dophenylether und deren bisher noch nicht beschriebenen Hydrolyse zur Gewinnung von 4,4'-Dihydroxidiphenylether stellen gegenüber den bisherigen Möglichkeiten zur Herstellung von 4,4'-Dihydroxidiphenylether zusammen einen technisch wesentlich besseren und wirtschaftlicheren Weg dar. Das ist einmal dadurch bedingt, daß bei der Halogenierung des Diphenylethers nach dem beanspruchten Verfahren kein Halogenwasserstoff als Reaktionsprodukt auftritt, weiter durch die bekannte leichtere Hydrolysierbarkeit von aromatischen Jodverbindungen mit wäßrigen Basen und insbesondere durch die quantitative Rückgewinnung des in das Verfahren eingebrachten Jods und, da hierbei in fester Form anfallend, leichte Abtrennbarkeit und Rückführung in die Jodierung des Diphenylethers. Bei dem erfindungsgemäßen Verfahren wird 4,4'-Dihydroxidiphenylether in Roh-Ausbeuten von über 90% d. Th., bezogen auf Diphenylether als Ausgangsprodukt, erhalten. Das erfindungsgemäße Verfahren zur Herstellung von 4,4'-Dihydroxidiphenylether ist durch die Verwendung des jodierten Zwischenprodukts anstelle des bromierten, mit üblichen Technologien gut beherrschbar.

Ausführung der Erfindung

Die oxidative Jodierung von Diphenylether wird in Gegenwart von Lösungsmitteln und in Gegenwart starker Säuren, wie z. B. Mineralsäuren, Trifluoressigsäure oder sogenannten Superacids als Katalysator durchgeführt. Als Lösungsmittel eignen sich die von Jodierungsreaktionen her bekannten Lösungsmittel, wie Alkohole, z. B. Methanol, oder Carbonsäuren, z. B. Essigsäure, denen zur Löslichmachung der Oxidationsmittel im Reaktionsmedium noch Wasser zugegeben wird. Auch für die abgestufte Löslichkeit der Reaktionsprodukte 4,4'-Dijoddiphenylether und Ammoniumhydrogensulfat bzw. Ammoniumsulfat, wie z. B. im Falle der Verwendung von Ammoniumpersulfat als Oxidationsmittel, und deren Abtrennung durch fraktionierte Kristallisation, ist ein darauf abgestimmtes Lösungsmittel-Wasser-Gemisch mit ca. 10 Gew.-% Wasser oder mehr vorteilhaft. Zur Säurekatalyse der Jodierungsreaktion ist als Mineralsäure sehr gut Schwefelsäure geeignet. Sie bringt eine hohe Reaktionsbeschleunigung und führt zu keinen Nebenreaktionen wie z. B. Salzsäure. Die Konzentration der Katalysatorsäure liegt bei 0.1 bis 10, insbesondere bei 1 bis 5 Gew.-% bezogen auf das Reaktionsmedium.

Die Reaktionstemparaturen liegen im Bereich von etwa Raumtemperatur bis ca. 100 °C, und die Reaktionszeiten, insbesondere von Reaktionstemperatur und Säurekonzentration abhängig, liegen im Bereich kleiner 1 Stunde bis mehrere Stunden. So wird Diphenylether in wäßriger Essigsäure unter Schwefelsäurekatalyse mit $J^2$ und Ammoniumpersulfat innerhalb einer Stunde in Dijoddiphenylether überführt, der bei der Reaktionstemperatur von 80 °C in dem Reaktionsmedium nicht löslich ist. Dieser wird heiß abgesaugt und das Filtrat auf Raumtemperatur abgekühlt. Dabei fällt das bei der Jodierung entstandene Ammoniumhydrogensulfat gegebenenfalls im Gemisch mit Ammoniumsulfat aus. Dieses wird abgesaugt und die Mutterlauge für eine erneute Jodierung eingesetzt.

Wird die Jodierung mit $H_2O_2$ als Oxidationsmittel, z. B. in wäßriger Essigsäure als Reaktionsmedium durchgeführt, so werden neben der Mineralsäure noch geringe Mengen Kupfer als Katalysatoren mitverwendet. Das Kupfer kann z. B. in Form eines Gemisches von Kupferpulver und Kupfer-(I)-chlorid zugesetzt werden oder kann aus kupferhaltigen Teilen der Umsetzungsapparatur stammen.

Das Jodierungsprodukt fällt dabei in hohen Ausbeuten als Gemisch von 4,4'-Dijoddiphenylether und 4-Hydroxi-4'-Jod-diphenylether, z. B. 1:1 molar, an, und kann als solches weiter in der Hydrolyse verwendet werden.

Die Hydrolyse von 4,4'-Dijoddiphenylether und/ oder 4-Hydroxi-4'-Jod-diphenylether

wird so durchgeführt, daß das aus der Jodierung stammende und mit Wasser gewaschene Produkt in wäßrig-alkalischem Medium, mit wasseranteilen von 10 bis 50 Gew.-%, unter Kupfer. bzw. Kupferionen- und Alkali- bzw. Erdalkaliperoxidkatalyse auf Temperaturen von etwa 100 bis etwa 200 °C erhitzt wird. Als Basen werden bei der Reaktion im wesentlichen starke anorganische Basen, insbesondere Alkali- und/oder Erdalkalihydroxide in Anteilen, die etwa der stöchiometrisch notwendigen bis zu 6fach stöchiometrischen Menge entsprechen, verwendet. Der Kupferkatalysator und der Peroxidkatalysator werden in Mengen von je 1 bis 10 Gew.-% bezogen auf den 4,4-Dijoddiphenylether eingesetzt.

Die nach der Reaktion abgekühlte und weiter mit Wasser verdünnte Reaktioslösung wird durch Filtration vom Kupfer befreit und anschliessend mit Säure, z. B. mit konzentrierter Salzsäure, unter Einstellung eines pH-Wertes $\leqslant 7$ versetzt. Dabei fällt der 4,4-Dihydroxidiphenylether aus und wird durch Filtration isoliert. Durch Umkristallisation aus Wasser wird reiner 4,4'-Dihydroxidiphenylether erhalten.

Die Jodregenerierung

lässt sich z. B. nach «Ullmanns Encyklopädie der technischen Chemie», 4. Auflage, Bd. 13, S. 423, durch Oxidation, mit z. B. Chlor, quantitativ durchführen, wobei das Jod als Festprodukt abgetrennt und in die Jodierung von Diphenylether zurückgeführt wird.

Die Erfindung wird durch die folgenden Beispiele näher erläutert.

Beispiel

Oxidative Jodierung von Diphenylether mit Ammoniumperoxodisulfat

In einem 2 l Dreihalskolben, der mit Rührer, Thermometer und Kühler versehen war, wurden 76,6 g (0,45 mol) Diphenylether und 125,2 g (0,49 mol) Jod in 700 ml 85%iger Essigsäure gelöst und 143,8 g (o,63 mol) Ammoniumperoxodisulfat sowie 25 ml konzentrierte Schwefelsäure hinzugegeben. Der Ansatz wurde langsam auf 80 °C hochgeheizt und 1 Stunde bei dieser Temperatur gehalten. Der ausgefallene 4,4'-Dijoddiphenylether wurde heiß abfiltiert und der Filterkuchen mit heißem Wasser und anschließend mit wenig kaltem Methanol gewaschen. Nach dem Erkalten des Filtrats wurde das ausgefallene Ammoniumhydrogesulfat abgesaugt und die Mutterlauge für weitere Jodierungen eingesetzt. Die Rohausbeute an 4,4'-Dijoddiphenylether betrug 198 g (96%). Nach Umkristallisation aus 2 l Eisessig wurden 137 g (72%) farblose Kristalle mit einem Schmelzpunkt von 138 °C (Lit. 141 °C) erhalten.

Oxidative Jodierung von Diphenylether mit Wasserstoffperoxid

In einem 1 l Vierhalskolben, der mit Rührer, Thermometer, Kühler und Tropftrichter versehen war, wurden 34,0 g (0,2 mol) Diphenylether und 55,7 g (0,22 mol) Jod in 270 ml Eisessig gelöst. Dazu wurden 2 g Kupferpulver, 1,5 g CuCl, 24 ml Wasser sowie 12 ml konzentrierte Schwefelsäure gegeben. Der Ansatz wurde auf 50 °C erwärmt und über den Tropftrichter 38,0 (0,28 mol) 25%ige Perhydrol-Lösung zugegeben. Dabei werden 10 g sofort zugegeben und der Rest innerhalb von 1 Stunde zugetropft. Es wurde 2 Stunden bei 50 °C gerührt. Nach dem Abkühlen wurde das ausgefallene Produkt abgesaugt und mit kaltem Methanol gewaschen. Es wurden 59,6 g (81% d. Th.) eines 1:1 Gemisches aus 4,4'-Dijoddiphenylether und 4-Hydroxi-4'-Joddiphenylether erhalten.

Hydrolyse

Herstellung von 4,4'-Dihydroxidiphenylether

In einen 0,5 l Dreihalskolben, der mit Rührer, Thermometer und Kugelkühler versehen war, wurden 84,4 g (0,2 mol) 4,4'-Dijoddiphenylether, 93,0 g (2,32 mol) NaOH, 2,1 g Kupferpulver, 1,7 g CuCl, 3,3 g Natriumperoxid sowie 33 ml Wasser 4 Stunden auf 190 °C erhitzt. Nach dem Abkühlen auf 140°C wurden weitere 37 ml Wasser dazugegeben und weitere 3 Stunden bei 150 °C gerührt. Nach dem Abkühlen auf Raumtemperatur wurde der Ansatz mit 200 ml Wasser versetzt und filtriert. Zum Filtrat wurden 200 g Eis gegeben und mit konzentrierter Salzsäure angesäuert. Der ausgefallene 4,4'-Dihydroxidiphenylether wurde abgesaugt und aus 0,8 l Wasser umkristallisiert. Ausbeute: 33,5 g (83%).

Jodrückgewinnung

Die saure Mutterlauge aus Beispiel 3 wurde mit 50 ml 30% Perhydrol versetzt und die ausgefallenen Jodkristalle abgesaugt. 46,2 g (91%) Jod konnten zurückgewonnen und in die oxidative Jodierung zurückgeführt werden.

**Patentansprüche**

1. Verfahren zur Herstellung von 4,4'-Dihydroxidiphenylether aus Diphenylether durch Iodierung des Diphenylethers und hydrolytischer Abspaltung des Iods, dadurch gekennzeichnet, daß die Iodierung des Diphenylethers in Gegenwart von Lösungsmitteln mit elementarem Jod und anorganischen Peroxoverbindungen als Oxidationsmittel in Gegenwart starker Säuren als Katalysatoren, wobei im Falle von $H_2O_2$ als Peroxoverbindung noch Katalysatoren von Schwermetallen, die in verschiedenen Oxidationsstufen vorkommen und leicht in diese überführbar sind, mitverwendet werden, bei Temperaturen von 20 bis 100 °C durchgeführt wird, anschließend der jodierte Diphenylether in wäßrig-alkalischem Medium in Gegenwart katalytischer Mengen von Kupfer bzw. Kupferionen und Alkali- bzw. Erdalkaliperoxid bei 100 bis 200 °C hydrolysiert wird und dann aus der filtrierten Lösung durch Säurezugabe unter Einstellung eines pH-Wertes ≤7 der 4,4'-Dihydroxidiphenylether freigesetzt wird.

2. Verfahren nach Anspruch 1, dadurch gekennzeichnet, dass die Iodierung mit Persulfaten und/oder Wasserstoffperoxid als anorganischen Peroxoverbindungen durchgeführt wird.

3. Verfahren nach den Ansprüchen 1 und 2, dadurch gekennzeichnet, dass die Iodierung mit Wasserstoffperoxid als Oxidationsmittel und katalytischen Mengen von Kupfer und/oder Kupferionen durchgeführt wird.

**Claims**

1. Process for the preparation of 4,4'-dihydroxydiphenylether from diphenylether by iodination of the diphenylether and hydrolytic cleavage of the iodine, characterised in that the iodination of the diphenylether is carried out of temperatures of 20 to 100 degrees C in the presence of solvents using elemental iodine and inorganic peroxo compounds as oxidation agents in the presence of strong acids as catalysts, wherein, in the case of $H_2O_2$ as the peroxo compound, further heavy metal catalysts, which occur in various oxidation states and can easily be converted into these various oxidation states, are co-used, then the iodinated diphenylether is hydrolysed at 100 to 200 degrees C in an aqueous alkaline medium in the presence of catalytic amounts of copper or copper ions and alkali peroxide or alkaline earth peroxide, and then the 4,4'-dihydroxydiphenylether is removed from the filtered solution by addition of acid while adjusting the pH value to ≤7.

2. Process according to claim 1, characterised in that the iodination is carried out using persulphates and/or hydrogen peroxide as inorganic peroxo compounds.

3. Process according to claims 1 and 2, characterised in that the iodination is carried out using hydrogen peroxide as the oxidising agent and catalytic amounts of copper and/or copper ions.

**Revendications**

1. Procédé de préparation de 4,4'-dihydroxydiphényléther à partir de diphényléther par iodation du diphényléther et élimination hydrolytique de l'iode, caractérisé en ce que l'iodation du diphényléther est conduite à des températures de 20 à 100 °C en présence de solvants, avec de l'iode élémentaire et des composés peroxo inorganiques servant d'agents d'oxydation et en présence d'acides forts servant de catalyseur, avec utilisation simultanée, en cas d'emploi de $H^2O^2$ comme composé peroxo, de catalyseurs de métaux lourds qui se présentent dans différents stades d'oxydation et qui peuvent être facilement transformés en ceux-ci, après quoi le diphényléther iodé est hydrolysé à 100—200 °C dans un milieu hydro-alcalin en présence de quantités catalytiques de cuivre ou d'ions cuivre et de peroxyde alcalin ou alcalino-terreux, puis le 4,4'-dihydroxydiphényléther est libéré de la solution filtrée par addition d'acide avec réglage d'un pH ≤7.

2. Procédé selon la revendication 1, caractérisé en ce que l'iodation est conduite avec des persulfates et/ou du peroxyde d'hydrogène en tant que composés peroxo inorganiques.

3. Procécé selon la revendication 1 ou 2, caractérisé en ce que l'iodation est conduite avec du peroxyde d'hydrogène en tant qu'agent d'oxydation et avec des quantités catalytiques de cuivre et/ou d'ions cuivre.